# EUROPEAN PATENT SPECIFICATION

(11) **EP 0 816 330 B1**
(45) Date of publication and mention of the grant of the patent: **27.12.2000**
(21) Application number: 97304190.8
(22) Date of filing: 16.06.1997
(51) Int. Cl.: C07C 233/76, C07D 213/82, A01N 43/40

(54) **Process for the preparation of N-acetonylbenzamides**
Verfahren zur Herstellung von N-Acetonylbenzamide
Procédé de préparation de N-acétonylbenzamides

(30) Priority: 28.06.1996 US 20516
(43) Date of publication of application: 07.01.1998
(73) Proprietor: ROHM AND HAAS COMPANY, Philadelphia, Pennsylvania 19106-2399 (US)
(72) Inventor: Michelotti, Enrique Luis, Fort Washington, Pennsylvania 19034 (US); Young, David Hamilton, Pennsylvania 19002 (US); McLaughlin, Thomas Anthony, Penndel, Pennsylvania 19047 (US)
(74) Representative: Buckley, Guy Julian

(56) References cited:
- EP-A- 0 170 498
- EP-A- 0 173 453
- EP-A- 0 600 629
- EP-A- 0 604 019
- TETRAHEDRON LETTERS, vol. 25, no. 31, 1984, OXFORD GB, pages 3277-3280, XP002041169 E.M. GORDON ET AL.:

## Description

This invention relates to new methods of preparing the N-acetonylbenzamides.

N-acetonylbenzamide fungicides are known, see, *e. g.*, U. S. Patent Nos. 5,254,584 and 5,304,572. One advantage of these known fungicides is that they have high fungicidal activity. Such compounds are particularly advantageous because their high activity allows them to be used at low application rates. However, there is always a need for fungicidal compounds of even higher activity. This results in lower use rates and, therefore, less environmental contamination.

We have discovered that with certain N-acetonylbenzamide fungicides which contain an assymetric carbon atom, the fungicidal activity results primarily from one enantiomer. Thus, fungicidal compositions containing only the active enantiomer provide higher fungicidal activity than compositions containing both enantiomers, when used at the same use rate.

This invention provides a process for preparing a compound of formula I 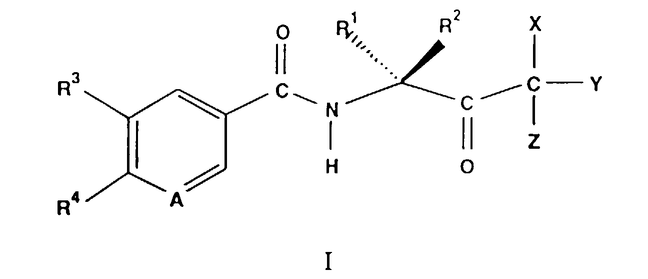 wherein:
1. A is selected from N and C-R⁵;
2. R¹ and R² are different and are independently selected from H, (C₁-C₆)alkyl, (C₂-C₆)alkenyl, (C₂-C₆)alkynyl, and halo(C₁-C₆)alkyl;
3. R³, R⁴, and R⁵ are independently selected from H, halo, (C₁-C₆)alkyl, (C₂-C₆)alkenyl, (C₂-C₆)alkynyl, halo(C₁-C₆)alkyl, (C₁-C₆)alkoxy, halo(C₁-C₆)alkoxy, cyano, nitro, -CR⁶=NOR⁷, -NR⁸R⁹, -CONR¹⁰R¹¹, and -NH-CO-OR¹² wherein R⁶ is selected from H, (C₁-C₆)alkyl, (C₂-C₆)alkenyl, and (C₂-C₆)alkynyl, R⁷ is selected from H, (C₁-C₆)alkyl, (C₂-C₆)alkenyl, (C₂-C₆)alkynyl, and (C₁-C₆)alkylcarbonyl, R⁸ and R⁹ are independently selected from H, (C₁-C₆)alkyl, and (C₁-C₆)alkylcarbonyl, R¹⁰ and R¹¹ are independently selected from H and (C₁-C₆)alkyl; and R¹² is selected from H, (C₁-C₆)alkyl, (C₂-C₆)alkenyl, and (C₂-C₆)alkynyl; and
4. X, Y, and Z are independently selected from H, halo, cyano, thiocyano, isothiocyano, and (C₁-C₄)alkylsulfonyloxy; provided that X, Y, and Z are not all H.

The term "halo" means chloro, fluoro, bromo, or iodo. The terms "alkyl" and "alkenyl" include straight-chain, branched-chain, and cycloalkyl and alkenyl groups. The term "alkynyl" includes straight-chain and branched-chain alkynyl groups. The term "alkoxy" includes as the alkyl portion straight-chain, branched-chain, and cyclic alkyl and alkenyl groups. The term "halo" preceeding any one of alkyl, alkenyl, alkynyl, or alkoxy means that one or more of the hydrogens of the group is substituted with a halogen.

The term "stereochemically larger" means the group in question is more space-filling than the group to which it is being compared. When the R¹ and R² groups in formula I contain only carbon and hydrogen atoms, since R² is the stereochemically larger group, the stereochemistry about the atom to which the R¹ and R² groups are attached will take on an "S" configuration. That is, the compound of formula I is designated as the S enantiomer. Throughout this application, the term "S enantiomer" means that the four groups on the carbon to which R¹ and R² are attached, when ranked according to the set of sequence rules of the Cahn-Ingold-Prelog system (*Angew. Chem. Int. Ed. Engl.* 5, 385-415(1966)), define the carbon as having an S configuration. The term "R enantiomer" means that the four groups form an R configuration. The term "predominantly free" means that the ratio of enantiomers is greater than 3:1, preferably greater than 5:1, more preferably greater than 10:1, and most preferably greater than 100:1.

Because of their high fungicidal activity preferred compounds are those of formula I wherein: R³ is selected from halo, cyano, nitro, and -CH=NOCH₃; R⁴ is selected from H, halo, cyano, (C₁-C₆)alkyl, -NH-CO-OR¹²,and -NR¹⁰R¹¹; R⁵ is selected from halo, cyano, and (C₁-C₆)alkyl; R¹ and R² are independently selected from (C₁-C₆)alkyl; X and Y are H; and Z is chloro.

Because of their outstanding fungicidal activity and selectivity the most preferred compounds of formula I are those wherein: R³ is selected from chloro, bromo, CN, and -CH=NOCH₃; R⁴ is selected from H, -NH₂, CN, and -CH₃; R⁵ is selected from chloro, bromo, CN, and -CH₃; R¹ is methyl; R² is ethyl; X and Y are H; and Z is chloro.

This invention provides a process for preparing compounds of formula I, comprising the steps of:
a. reacting a protonated amino acid ester of the formula: 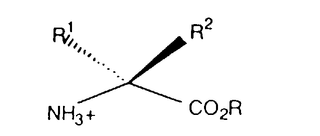 wherein R¹ and R² are different and are independently selected from H, (C₁-C₆)alkyl, (C₂-C₆)alkenyl, (C₂-C₆)alkynyl, and halo(C₁-C₆)alkyl and R is selected from (C₁-C₆)alkyl,
   with an acyl chloride of the formula: 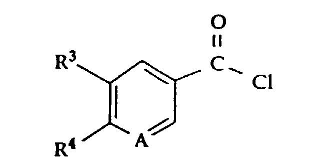 wherein A is selected from N and C-R⁵ and R³, R⁴, and R⁵ are independently selected from H, halo, (C₁-C₆)alkyl, (C₂-C₆)alkenyl, (C₂-C₆)alkynyl, halo(C₁-C₆)alkyl, (C₁-C₆)alkoxy, halo(C₁-C₆)alkoxy, cyano, nitro, -CR⁶=NOR⁷, -NR⁸R⁹, -CONR¹⁰R^{11,} and -NH-CO-OR¹² wherein R⁶ is selected from H, (C₁-C₆)alkyl, (C₂-C₆)alkenyl, and (C₂-C₆)alkynyl, R⁷ is selected from H, (C₁-C₆)alkyl, (C₂-C₆)alkenyl, (C₂-C₆)alkynyl, and (C₁-C₆)alkylcarbonyl, R⁸ and R⁹ are independently selected from H, (C₁-C₆)alkyl, and (C₁-C₆)alkylcarbonyl, R¹⁰ and R¹¹ are independently selected from H and (C₁-C₆)alkyl; and R¹² is selected from H, (C₁-C₆)alkyl, (C₂-C₆)alkenyl, and (C₂-C₆)alkynyl;
   to produce a benzamide-ester of the formula: 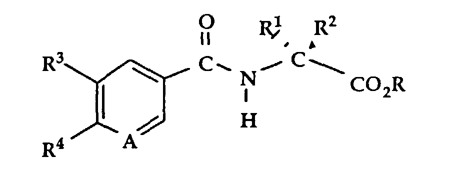
b. hydrolyzing the ester moiety of the benzamide-ester to produce a benzamide-acid of the formula: 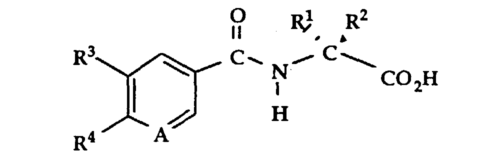
c. cyclizing the benzamide-acid to produce an oxazolinone of the formula: 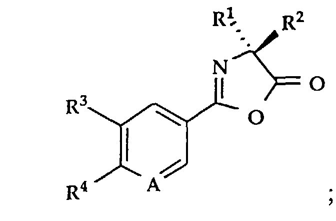 and
d. forming the compound of formula I by ring opening the oxazolinone.

The protonated amino acid ester may be prepared using standard esterification procedures such as treatment of the corresponding amino acid with an alcohol under acidic conditions. We have found that methanol is the preferred alcohol because of the ease of removal of a methyl group during the hydrolyzing step.

In a similar manner, the hydrolyzing step is conducted using standard conditions. Base catalyzed hydrolysis using sodium hydroxide as the base is preferred. The only limitations to the reaction conditions used in the hydrolyzing step are that the conditions must be sufficiently selective so that the ester bond is hydrolyzed but the amide bond is not. Strong base catalysts must be avoided when R¹ or R² is hydrogen to eliminate side reactions resulting from abstraction of the hydrogens.

The oxazolinone is produced in the cyclization step by dehydration of the benzamide-acid. Such dehydrations may be conducted using a variety of dehydrating agents such as acetic anhydride at elevated temperatures (90°-100° C), phosphorous oxychloride, phosphorous pentachloride, and ethyl chloroformate/triethylamine. Mild dehydrating agents such as acetic anhydride are preferred because they are easily removed and side reactions are avoided.

Ring opening of the oxazolinone to form the compound of formula I may be conducted in a single or in multiple steps. A single step ring opening is treating the oxazolinone with chloromethyllithium which produces the compound of formula I wherein X and Y are H and Z is Cl. A multiple step ring opening is treating the oxazolinone first with methyllithium to form the compound of formula I wherein X, Y, and Z are all H, chlorinating the ketone to produce a mixture of compounds of formula I wherein one or two of X, Y, and Z are Cl and the remaining are H, followed by selective removal of one chlorine atom from any compound in which two of X, Y, and Z are Cl to give a compound of formula I wherein two of X, Y, and Z are H and the remaining is Cl. The removal of one chlorine atom may be accomplished by hydrogenation of the dichloro compound in the presence of a catalyst such as palladium.

This same process may also be employed to produce a racemic mixture of R and S isomers of the compound of formula I by utilizing a racemic mixture of the R and S isomers of the protonated amino acid ester in the first step.

Compositions containing compounds of formula I and an agronomically acceptable carrier are useful in controlling a broad spectrum of phytopathogenic fungi such as those of the classes Oomycetes, Deuteromycetes, and Ascomycetes.

The compositions containing the compounds prepared by the present invention (compounds of formula I) are useful for the control of phytopathogenic fungi on crops and may be used as seed protectants, soil fungicides and/or foliar fungicides. As a seed protectant, a compound of the present invention is coated on seed at a dosage rate of about 5 grams (g) compound per 50 kilograms (kg) seed to about 250 g compound per 50 kg seed. As a soil fungicide, a compound of the present invention can be incorporated in the soil or applied to the surface of the soil at a dosage rate of about 0.25 kg compound per hectare to about 10 kg compound per hectare and preferably at a rate of about 0.5 k compound per hectare to about 2.5 kg compound per hectare.

The compositions containing the compounds prepared by of the present invention can be applied to plant foliage as fungicidal sprays by methods commonly employed, such as conventional high-gallonage hydraulic sprays, low-gallonage sprays, air-blast, aerial sprays and dusts. While the dilution and rate of application will depend upon the type of equipment employed, the method and frequency of application desired and diseases to be controlled, the effective amount is typically from about 0.005 kg compound per hectare to about 1.0 kg compound per hectare, preferably from about 0.05 kg compound per hectare to about 0.5 kg compound per hectare and more preferably from about 0.0625 kg compound per hectare to about 0.25 kg compound per hectare.

For the above disclosed purposes these compounds can be used in the pure form, also known as technical in the art, as prepared, or as solutions or as formulations. The compounds are usually provided with a carrier or are formulated so as to render them suitable for subsequent use as fungicides. For example, the compounds can be formulated as wettable powders, dry powders, emulsifiable concentrates, dusts, granular formulations, aerosols, or flowable emulsion concentrates. In such formulations, the compounds are extended with a liquid or solid carrier and, when dried, suitable surfactants are incorporated.

It is usually desirable, particularly in the case of foliar spray formulations, to include adjuvants, such as wetting agents, spreading agents, dispersing agents, stickers, adhesives and the like in accordance with agricultural practices. Such adjuvants commonly used in the art can be found in McCutcheon's Emulsifiers and Detergents, McCutcheon's Emulsifiers and Detergents/Functional Materials and McCutcheon's Functional Materials all published annually by McCutcheon Division of MC Publishing Company (New Jersey).

In general, the compounds utilized in this invention can be dissolved in appropriate solvents such as acetone, methanol, ethanol, dimethylformamide or dimethyl sulfoxide and such solutions extended with water. The concentrations of the solution can vary from 1% to 90% with a preferred range being 5% to 50%.

For the preparation of emulsifiable concentrates, the compounds used in the invention can be dissolved in suitable organic solvents or a mixture of solvents, together with an emulsifying agent which permits dispersion of the fungicide in water. The concentration of the active ingredient in emulsifiable concentrates is usually 10% to 90% and in flowable emulsion concentrates, this can be as high as 75%. Wettable powders suitable for spraying, can be prepared by admixing the compound with a finely divided solid or mixture of solids, such as clays, inorganic silicates, inorganic carbonates, and silicas and incorporating wetting agents, sticking agents, and/or dispersing agents in such mixtures. The concentration of active ingredients in such formulations is usually in the range of 20% to 98%, preferably 40% to 75%.

Dusts are prepared by mixing the compounds of the present invention salts and complexes thereof with finely divided inert solids which can be organic or inorganic in nature. Inert materials useful for this purpose include botanical flours, silicas, silicates, carbonates and clays. One convenient method of preparing a dust is to dilute a wettable powder with a finely divided carrier. Dust concentrations containing 20% to 80% of the active ingredient are commonly made and are subsequently diluted to 1% to 10% use concentration.

The compounds of the present invention can also be utilized in combination with other fungicides such as, for example, those disclosed in U. S. Patent No. 5,304,572 (column 3, line 30 to column 4, line 52) as well as acylalanines such as , furalaxyl, cyprofuram, ofurace, benalaxyl, and oxadixyl;, fluazinam, flumetover, phenylbenzamide derivatives such as those disclosed in EP 578586 A1, amino acid derivatives such as valine derivatives disclosed in EP 550788 A1, methoxyacrylates such as methyl (E)-2-(2-(6-(2-cyanophenoxy)pyrimidin-4-yloxy)phenyl)-3-methoxyacrylate; benzo(1,2,3)thiadiazole-7-carbothioic acid S-methyl ester: propamocarb; imazalil; carbendazim; myclobutanil; fenbuconazole; tridemorph; pyrazophos; fenarimol; fenpiclonil; pyrimethanil; and tin fungicides. Those skilled in the art will recognize that mixtures of the respective compositions and compounds of the present invention with other fungicidally active compounds may provide advantages such as a broader spectrum of antifungal activity than the respective compositions and compounds of the present invention alone.

In a similar manner, the compositions and compounds of this invention may be applied in combination with one or more insecticides such as those disclosed in U. S. Patent No. 5,075,471 (columns 14 and 15). Again, those skilled in the art will recognize that mixtures of the respective compositions and compounds of the present invention with insecticidally active compounds may provide advantages such as fewer total applications than if the fungicides and insecticides are applied separately.

The following examples describe in detail some of the embodiments of this invention.

### Methods of Preparation

### Preparation of racemic isovaline [(R,S) 2-amino-2-methylbutanoic acid].

The preparation of this compound was carried out by a modified procedure from Chirality (1992) 4, 302-7.

A 2-liter stainless steel autoclave containing 5-ethyl-5-methylhydantoin (Frinton Labs) (100.0 g, 0.70 mole), barium hydroxide octahydrate (440 g, 1.395 mole) and deionized water (1.25 L) was sealed and heated to 175 °C for 15 hours. The cooled reaction mixture was filtered through diatomaceous earth (Celite). The resulting white cake was washed thoroughly with deionized water. The combined aqueous filtrate and water washings were treated with carbon dioxide gas (from 120 g of dry ice). The solid formed was separated by filtration and the clear aqueous solution was concentrated in the rotary evaporator until the wet solids coated the sides of the flask. The resulting suspension was triturated with a mixture of 1:1 acetone:ethanol (300 ml) to afford a white solid that after drying yielded 71.5 g (87.3%) of the expected racemic isovaline.

### Preparation of N-chloroacetyl isovaline.

### Procedure adapted from J. Amer. Chem. Soc. 4701 (1952).

To a well-stirred mixture chilled to 0 °C to 5 °C (ice bath) of racemic isovaline ((R,S) 2-amino-2-methylbutanoic acid) (350 g, 2.99 mole) and 2N aqueous sodium hydroxide (1.5 L) were added simultaneously chloroacetyl chloride (373 g, 3.31 mole) and 2N aqueous sodium hydroxide (1,718 ml, 3.44 mole) over 1.5 hours . The base was added at such a rate as to keep the reaction mixture basic at all times. The reaction mixture was warmed up to room temperature, treated with concentrated aqueous hydrochloric acid until acidic to litmus paper. A white solid formed which was separated by filtration and dried to yield 454 g (78.5%) of the expected racemic N-chloroacetylisovaline ((R,S) 2-chloroacetamido-2-methylbutanoic acid).

### Enzymatic resolution of racemic N-chloroacetylisovaline [(R,S) 2-chloroacetamido-2-methylbutanoic acid].

Adapted from J. Amer. Chem. Soc. 4701 (1952) and Chemistry of Amino Acids Volume 3 page 2575. John Wiley and Sons Edited by J.P.Greensteins and M. Winitz.

Racemic N-chloroacetylisovaline ((R,S) 2-chloroacetamido-2-methylbutanoic acid) (120 g, 0.62 mole) was suspended in purified deionized water (1 L) and brought into solution by the addition of 2N aqueous sodium hydroxide to a pH of 7.5. Acylase I powder 75% (Sigma Chemicals Catalog Number A-3010) (1 g) was added and the pH was adjusted to 7.5. The resulting mixture was digested at 38 °C for 72 to 96 hours. The pH of the reaction mixture was adjusted to 5, and the resulting mixture stirred at 95 °C for approximately 2 hours. The aqueous mixture was filtered yielding a clear, slightly yellow solution. A total of 5 batches were run under the same conditions. All the batches were combined and divided in three. Each of these three batches was poured into a Dowex 50 (H⁺) column (1.75 L of wet resin) and washed with water until the pH of the eluate was greater than 5. The eluate was concentrated yielding (R)-N-chloroacetylisovaline. The (S)-isovaline on the Dowex 50 resin column was eluted with 2.5N aqueous hydrochloric acid (approximately 4 L). The combined acidic eluate was concentrated *in vacuo*. The resulting white solid was vacuum dried yielding a total of 355 g of a mixture of (S)-isovaline hydrochloride and sodium chloride used as such in the next step.

### Preparation of (S)-isovaline methyl ester hydrochloride [Methyl (S)-2-amino-2-methylbutanoate]

To a well-stirred suspension of the previous mixture of (S)-isovaline hydrochloride and sodium chloride in methanol (3 L) was slowly added thionyl chloride (373 g, 3.13 mole). After the addition was complete the reaction mixture was refluxed for 3 hours. The resulting mixture was cooled to room temperature and filtered. The resulting white filter cake was washed several times with methanol. The combined methanol filtrate and washings were concentrated using a rotary evaporator. Toluene was added to the resulting crude residue and then removed using the rotary evaporator yielding 187 g of the expected (S)-isovaline methyl ester hydrochloride.

### Preparation of (S)-N-(3,5-dichloro-4-methylbenzoate) isovaline methyl ester

In a 5-liter round-bottomed flask were placed the previously prepared (S)-isovaline methyl ester hydrochloride (280 g, 1.67 mole), 3,5-dichloro-4-methylbenzoyl chloride (381 g, 1.705 mole) and methylene chloride (2.2 L). The mixture was cooled to 0 °C. To the resulting cooled (0 °C) mixture was added slowly triethylamine (540 ml) keeping the reaction mixture at 0 °C. When the addition was complete the reaction mixture was stirred at 0 °C for 30 minutes and then allowed to warm up to room temperature. The reaction mixture was washed sequentially with water, 2% aqueous hydrochloric acid, water, saturated aqueous sodium bicarbonate and finally brine. The organic layer was dried over anhydrous magnesium sulfate and solvent eliminated using a rotary evaporator yielding 505.9 g of the expected (S) N-(3,5-dichloro-4-methylbenzoate) isovaline methyl ester which was used as such in the next step.

### Preparation of (S)-N-(3,5-dichloro-4-methylbenzoate) isovaline .

To a mixture of the previously prepared (S)-N-(3,5-dichloro-4-methylbenzoate) isovaline methyl ester (315 g) and methanol (3 L) at 55 °C was added slowly aqueous sodium hydroxide (10% solution, 869 g, 2.17 mole). When the addition was complete the reaction mixture was refluxed for 1 hour. The reaction mixture was cooled to room temperature and the solvent eliminated using a rotary evaporator. The crude reaction product was taken up in water, the resulting aqueous solution was washed 3 times with ethyl acetate, and made acidic with concentrated aqueous hydrochloric acid. The product settled first as an oil which quickly solidified. The solids were separated by filtration, washed several times with water and dried in a vacuum oven yielding 279 g of the expected (S) N-(3,5-dichloro-4-methylbenzoate) isovaline which was used as such in the next step.

### Preparation of (S)-2-(3,5-dichloro-4-methylbenzoyl)-4-ethyl-4-methyl-1,3-oxazol-5-one.

A mixture of the previously prepared (S)-N-(3,5-dichloro-4-methylbenzoate) isovaline (279 g, 0.917 mole) and acetic anhydride (1.25 L) was refluxed for 1 hour. The reaction mixture was cooled to room temperature and the solvent was eliminated in the rotary evaporator yielding a thick oily residue. This residue was treated with xylene and the solvent was eliminated using a rotary evaporator. The resulting crude product was dried in a vacuum oven yielding 275.5 g of the expected (S)-2-(3,5-dichloro-4-methylbenzoyl)-4-ethyl-4-methyl-1,3-oxazol-5-one as an oil that quickly solidified. The compound was used as such in the next step.

### Preparation of (S)-N-(1-ethyl-1-methyl-2-oxopropyl)-3,5-dichloro-4-methylbenzamide

In a 3-liter four-necked round-bottomed flask equipped with mechanical stirrer, condenser with nitrogen inlet on top, thermometer, and addition funnel were placed the previously prepared 2-(3,5-dichloro-4-methylbenzoyl)-4-ethyl-4-methyl-1,3-oxazol-5-one (107 g, 0.374 mole) and dry tetrahydrofuran (1.4 L). To the resulting mixture cooled down to -70 °C was added slowly dropwise methyllithium (1.4 M solution, 280 ml, 0.392 mole) over 20 minutes period. After the addition was complete the reaction mixture was warmed up to room temperature and poured into a saturated aqueous solution of ammonium chloride. The organic phase was separated and the aqueous layer was extracted three times with ethyl acetate. The combined organic layers were dried over anhydrous magnesium sulfate and the solvent eliminated in the rotary evaporator yielding 117.3 g of the expected (S)-N-(1-ethyl-1-methyl-2-oxopropyl)-3,5-dichloro-4-methylbenzamide as a thick oil.

### Preparation of (S)-N-(3-chloro-1-ethyl-1-methyl-2-oxopropyl)-3,5-dichloro-4-methylbenzamide and (S)-N-(3,3-dichloro-1-ethyl-1-methyl-2-oxopropyl)-3,5-dichloro-4-methylbenzamide

In a 2-liter four-necked round-bottomed flask equipped with mechanical stirrer, condenser with inlet on top connected to an acid scrubber, thermometer, and gas inlet tube were placed the previously prepared (S)-N-(1-ethyl-1-methyl-2-oxopropyl)-3,5-dichloro-4-methylbenzamide (115 g, 0.38 mole) and glacial acetic acid (1 L). The resulting mixture was warmed up to 60 °C and chlorine gas was admitted into the well-stirred reaction mixture. Chlorine was bubbled in until thin layer chromatography showed no more starting material. The reaction mixture was cooled down to room temperature and the solvent eliminated in the rotary evaporator yielding the crude product. This residue was triturated with hexane and filtered yielding 121.2 g of a mixture of (S)-N-(3-chloro-1-ethyl-1-methyl-2-oxopropyl)-3,5-dichloro-4-methylbenzamide and (S)-N-(3,3-dichloro-1-ethyl-1-methyl-2-oxopropyl)-3,5-dichloro-4-methylbenzamide which was used as such in the next step.

### Preparation of (S)-N-(3-chloro-1-ethyl-1-methyl-2-oxopropyl)-3,5-dichloro-4-methylbenzamide

The mixture (87 g) prepared in the previous step, 1.35 L of ethanol and 800 mg of 5% palladium over charcoal were placed in a hydrogenation bottle and hydrogenated in a Parr apparatus (50 psi, room temperature) for 3 hours. The reaction mixture was filtered through Celite and the solvent eliminated under reduced pressure, to yield a crude product. The crude product was triturated with hexane and filtered yielding after drying 56.6 g of the expected (S)-N-(3-chloro-1-ethyl-1-methyl-2-oxopropyl)-3,5-dichloro-4-methylbenzamide methylbenzamide (mp. 154-155° C, [α]_{D} = -4.1 in ethanol).

### Preparation of (R)-isovaline methyl ester

In a 2-liter round-bottomed flask equipped with a condenser and a magnetic stirrer were placed 70 g of the (R)-N-chloroacetylisovaline obtained from the enzymatic resolution of racemic N-chloroacetylisovaline, 696 ml of water, and 696 ml of concentrated hydrochloric acid. The resulting mixture was heated at reflux for 2.25 hours. The mixture was then cooled to room temperature and the solvent was removed using rotary evaporation yielding a solid residue. the residue was washed with dry acetone and then dried in a vacuum oven at 40° C yielding a crude product. The crude product was esterified with methanol using the above-described procedure for preparation of (S)-isovaline methyl ester hydrochloride to give 47.52 g of the corresponding (R)-isovaline methyl ester hydrochloride.

The (R)-isovaline methyl ester hydrochloride may be converted to (R)-N-(3-chloro-1-ethyl-1-methyl-2-oxopropyl)-3,5-dichloro-4-methylbenzamide (mp. 155.5-156° C, [α]_{D} = +4.14 in ethanol) using the above-described sequence for preparation of the (S)-enantiomer.

## Claims

1. A process for preparing a compound of the formula: 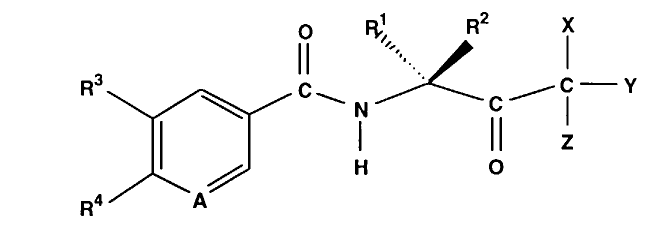 wherein:
R¹ and R² are different and are independently selected from H, (C₁-C₆)alkyl, (C₂-C₆)alkenyl, (C₂-C₆)alkynyl, and halo(C₁-C₆)alkyl;
A is selected from N and C-R⁵ and R³, R⁴, and R⁵ are independently selected from H, halo, (C₁-C₆)alkyl, (C₂-C₆)alkenyl, (C₂-C₆)alkynyl, halo(C₁-C₆)alkyl, (C₁-C₆)alkoxy, halo(C₁-C₆)alkoxy, cyano, nitro, -CR⁶=NOR⁷, - NR⁸R⁹, -CONR¹⁰R¹¹, and -NH-CO-OR¹² wherein R⁶ is selected from H, (C₁-C₆)alkyl, (C₂-C₆)alkenyl, and (C₂-C₆)alkynyl, R⁷ is selected from H, (C₁-C₆)alkyl, (C₂-C₆)alkenyl, (C₂-C₆)alkynyl, and (C₁-C₆)alkylcarbonyl, R⁸ and R⁹ are independently selected from H, (C₁-C₆)alkyl, and (C₁-C₆)alkylcarbonyl, R¹⁰ and R¹¹ are independently selected from H and (C₁-C₆)alkyl; and R¹² is selected from H, (C₁-C₆)alkyl, (C₂-C₆)alkenyl, and (C₂-C₆)alkynyl;
X, Y, and Z are independently selected from H and Cl; provided that one or two of X, Y, and Z is Cl;
comprising the steps of:
a. reacting a protonated amino acid ester of the formula: 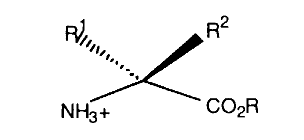 wherein R represents (C₁-C₆)alkyl with an acyl chloride of the formula: 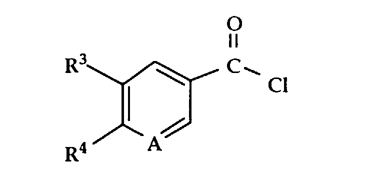 to produce a benzamide-ester of the formula: 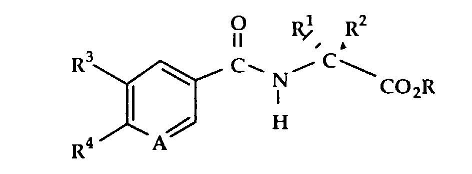
b. hydrolyzing the ester moiety of the benzamide-ester to produce a benzamide-acid of the formula: 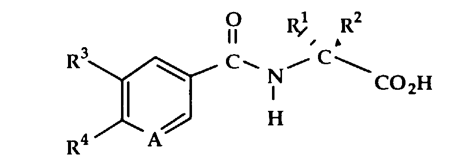
c. cyclizing the benzamide-acid to produce an oxazolinone of the formula: 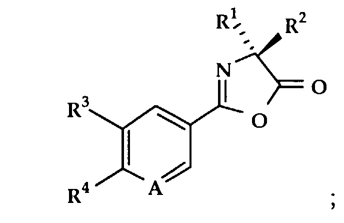 and
d. forming the compound of the formula: 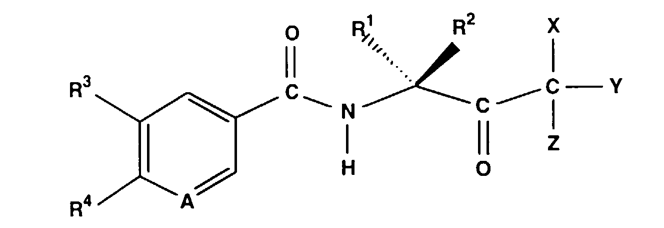 by ring opening the oxazolinone in a single or multiple-step process comprising:
i) treating the oxazolinone with chloromethyl lithium to produce a compound of formula I wherein X and Y are H and Z is Cl; or
ii) treating the oxazolinone first with methyllithium to form the compound of formula I wherein X, Y, and Z are all H and then selectivly replacing one or more of the hydrogens with chlorine.

## Patentansprüche

1. Ein Verfahren zur Herstellung einer Verbindung der Formel 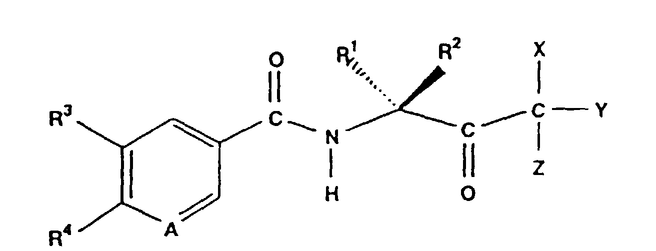 worin:
R¹ und R² verschieden sind und unabhängig voneinander ausgewählt sind aus H, (C₁-C₆)Alkyl, (C₂-C₆)Alkenyl, (C₂-C₆)Alkynyl und Halo(C₁-C₆)alkyl,
A ausgewählt ist aus N und C-R⁵ und R³, R⁴ und R⁵ unabhängig voneinander ausgewählt sind aus H, Halo, (C₁-C₆)Alkyl, (C₂-C₆)Alkenyl, (C₂-C₆)Alkynyl und Halo(C₁-C₆)alkyl, (C₁-C₆)Alkoxy, Halo(C₁-C₆)alkoxy, Cyano, Nitro, -CR⁶=NOR⁷, -NR⁸R⁹, -CONR¹⁰R¹¹ und -NH-CO-OR¹², worin R⁶ ausgewählt ist aus H, (C₁-C₆)Alkyl, (C₂-C₆)Alkenyl und (C₂-C₆)Alkynyl, R⁷ ausgewählt ist aus H, (C₁-C₆)Alkyl, (C₂-C₆)Alkenyl, (C₂-C₆)Alkynyl und (C₁-C₆)Alkylcarbonyl, R⁸ und R⁹ unabhängig voneinander ausgewählt sind aus H, (C₁-C₆)Alkyl und (C₁-C₆)Alkylcarbonyl, R¹⁰ und R¹¹ unabhängig voneinander ausgewählt sind aus H und (C₁-C₆)Alkyl, und R¹² ausgewählt ist aus H, (C₁-C₆)Akyl, (C₂-C₆)Alkenyl und (C₂-C₆)Alkynyl,
X, Y und Z unabhängig voneinander ausgewählt sind aus H und Cl, vorausgesetzt, dass ein oder zwei Reste von X, Y und Z Cl sind,
umfassend die Schritte:
a) Umsetzen eines protonierten Aminosäureesters der Formel: 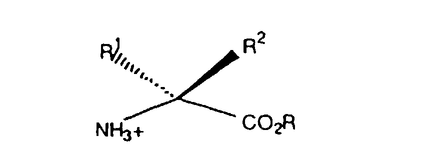 worin R ein (C₁-C₆)Alkyl ist, mit einem Acylchlorid der Formel 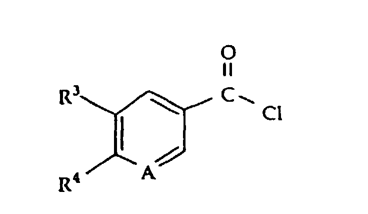 um einen Benzamidester der Formel: 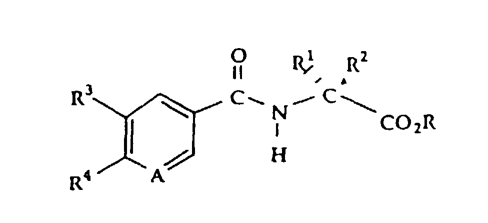 zu erzeugen,
b) Hydrolisieren der Esterkomponente des Benzamidesters, um eine Benzamidsäure der Formel: 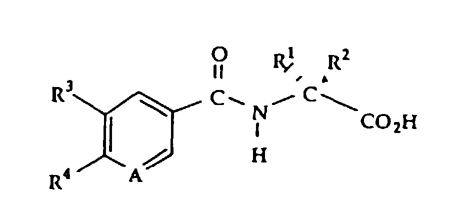 zu erzeugen,
c) Zyklisieren der Benzamidsäure, um ein Oxazolinon der Formel: 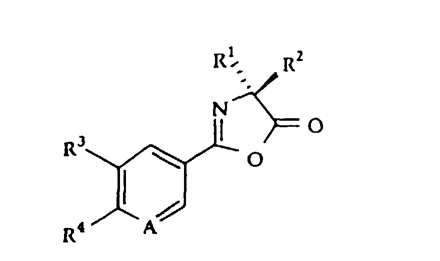 zu erzeugen, und
d) Bilden der Verbindung der Formel: 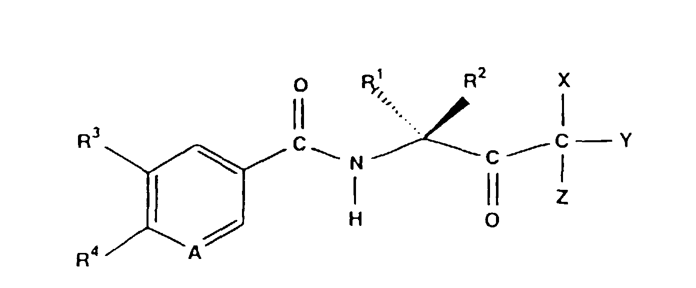 durch Ringöffnen des Oxazolinons in einem Einschritt- oder Mehrschrittprozeß, umfassend:
i) Behandeln des Oxazolinons mit Chlormethyllithium, um eine Verbindung der Formel I zu erzeugen, worin X und Y H sind und Z Cl ist, oder
ii) Behandeln des Oxazolinons zuerst mit Methyllithium, um die Verbindung der Formel I zu bilden, worin X, Y und Z H sind, und dann selektives Ersetzen eines oder mehrerer der Wasserstoffe durch Chlor.

## Revendications

1. Procédé de préparation d'un composé de formule: 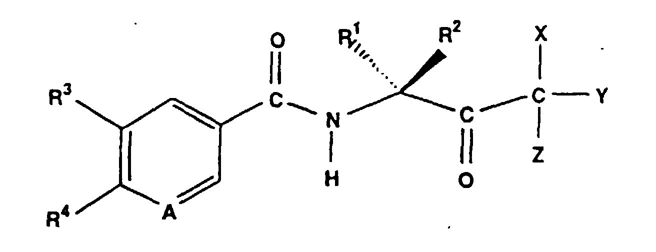 dans laquelle:
R¹ et R² sont différents et sont choisis indépendamment parmi H et les groupes alkyle en C₁-C₆, alcényle en C₂-C₆, alcynyle en C₂-C₆ et halogénoalkyle en C₁-C₆;
A est choisi parmi N et C-R⁵; et R³, R⁴ et R⁵ sont choisis indépendamment parmi H et les groupes halogéno, alkyle en C₁-C₆, alcényle en C₂-C₆, alcynyle en C₂-C₆, halogénoalkyle en C₁-C₆, alcoxy en C₁-C₆, halogénoalcoxy en C₁-C₆, cyano, nitro, -CR⁶=NOR⁷, -NR⁸R⁹, -CONR¹⁰R¹¹ et -NH-CO-OR¹², où R⁶ est choisi parmi H et les groupes alkyle en C₁-C₆, alcényle en C₂-C₆ et alcynyle en C₂-C₆, R⁷ est choisi parmi H et les groupes alkyle en C₁-C₆, alcényle en C₂-C₆, alcynyle en C₂-C₆ et alkyl(en C₁-C₆)carbonyle, R⁸ et R⁹ sont choisis indépendamment parmi H et les groupes alkyle en C₁-C₆ et alkyl(en C₁-C₆)carbonyle, R¹⁰ et R¹¹ sont choisis indépendamment parmi H et les groupes alkyle en C₁-C₆; et R¹² est choisi indépendamment parmi H et les groupes alkyle en C₁-C₆, alcényle en C₂-C₆ et alcynyle en C₂-C₆;
X, Y et Z sont choisis indépendamment parmi H et Cl; à condition qu'un ou deux des radicaux X, Y et Z soient Cl;
comprenant les étapes consistant à:
a. faire réagir un ester d'acide aminé protoné de formule: 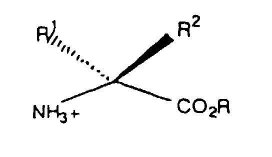 dans laquelle R représente un groupe alkyle en C₁-C₆, avec un chlorure d'acyle de formule: 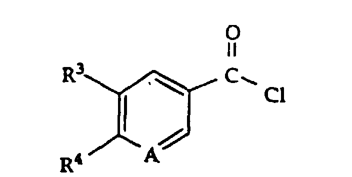 pour produire un benzamide-ester de formule: 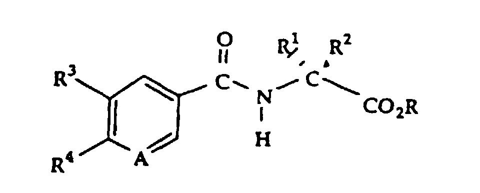
b. hydrolyser le groupement ester du benzamide-ester pour produire un benzamide-acide de formule: 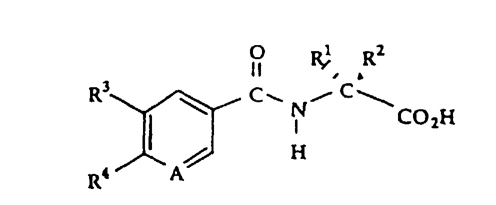
c. cycliser le benzamide-acide pour produire une oxazolinone de formule: 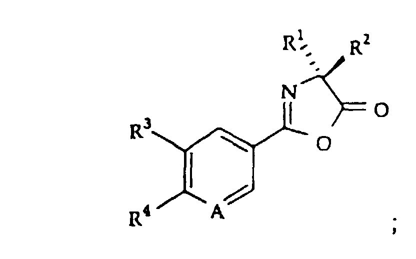 et
d. former le composé de formule: 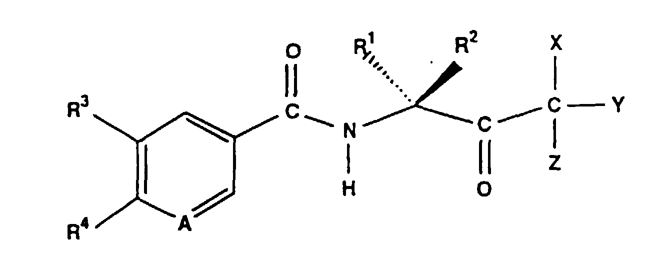 par ouverture de cycle de l'oxazolinone, dans un procédé en une seule étape ou en plusieurs étapes consistant
i) à traiter l'oxazolinone avec du chlorométhyllithium pour produire un composé de formule I, dans laquelle X et Y sont H et Z est Cl; ou
ii) à traiter l'oxazolinone d'abord avec du méthyllithium pour former le composé de formule I, dans laquelle X, Y et Z sont tous H, puis à remplacer sélectivement un ou plusieurs des atomes d'hydrogène par des atomes de chlore.
